# EUROPÄISCHE PATENTSCHRIFT

(11) **EP 0 651 913 B1**
(45) Veröffentlichungstag und Bekanntmachung des Hinweises auf die Patenterteilung: **02.10.1996**
(21) Anmeldenummer: 93915667.5
(22) Anmeldetag: 22.07.1993
(51) Int. Cl.: H01L 51/00, C07K 17/02

(54) **ANORDNUNG FÜR BIOELEKTRISCHE ANWENDUNGEN**
ARRANGEMENT FOR BIOELECTRICAL APPLICATIONS
DISPOSITIFS POUR APPLICATIONS BIOELECTRIQUES

(30) Priorität: 23.07.1992 DE 4224602
(43) Veröffentlichungstag der Anmeldung: 10.05.1995
(73) Patentinhaber: INSTITUT FÜR BIOPROZESS- UND ANALYSENMESSTECHNIK e.V., D-37308 Heiligenstadt (DE)
(72) Erfinder: BECKMANN, Dieter, D-37308 Heiligenstadt (DE); GRUBER, Ronald, D-37308 Geisleden (DE)
(74) Vertreter: Maikowski, Michael, Dipl.-Ing. Dr.
(86) Internationale Anmeldenummer: DE9300662
(87) Internationale Veröffentlichungsnummer: WO9402963

(56) Entgegenhaltungen:
- EP-A- 0 417 541
- US-A- 4 804 834
- DATABASE INSPEC INSTITUTE OF ELECTRICAL ENGINEERS, STEVENAGE, GB Inspec No. 867205 R. H. LOZIER ET AL. 'BACTERIORHODOPSIN: A LIGHT-DRIVEN PROTON PUMP IN HALOBACTERIUM HALOBIUM'
- SCIENCE Bd. 255 , 17. Januar 1992 , LANCASTER, PA US Seiten 342 - 344 T. MIYASAKA ET AL. 'QUANTUM CONVERSION AND IMAGE DETECTION BY A BACTERIORHODOPSIN-BASED ARTIFICIAL PHOTORECEPTOR'
- PATENT ABSTRACTS OF JAPAN vol. 14, no. 468 (P-1115)12. Oktober 1990 & JP,A,02 186 253 (AGENCY OF IND. SCIENCE & TECHNOL.) 20. Juli 1990
- PATENT ABSTRACTS OF JAPAN vol. 9, no. 62 (P-342)19. März 1985 & JP,A,59 197 849 (MITSUBISHI DENKI K.K.) 9. November 1984
- PATENT ABSTRACTS OF JAPAN vol. 12, no. 237 (C-509)6. Juli 1988 & JP,A,63 028 390 (SUNTORY LTD.) 6. Februar 1988
- INTERNATIONAL CONFERENCE OF THE IEEE ENGINEERING IN MEDICINE AND BIOLOGY SOCIETY Bd. 11 , 9. November 1989 , SEATTLE, USA Seiten 1333 - 1335 S. MICHAILE ET AL. 'SIGNAL MODULATION VIA INTERFACIAL PROCESSES IN MOLECULAR OPTOELECTRONIC DEVICES'
- PATENT ABSTRACTS OF JAPAN vol. 13, no. 47 (P-822)3. Februar 1989 & JP,A,63 241 432 (SANYO ELECTRIC CO. LTD.) 6. Oktober 1988 in der Anmeldung erwähnt

## Beschreibung

Die Erfindung bezieht sich auf eine Anordnung für bioelektrische Anwendungen, die als Strahlungssensor zum Nachweis unterschiedlicher Strahlungsarten und -intensitäten, zum Nachweis solcher Verbindungen, die das eingeschlossene biologische Material in seiner Strahlungsempfindlichkeit beeinflussen, und als Solarbatterie verwendet wird.

Es ist eine bioelektrische Anordnung bekannt, bei der ein photoempfindliches Protein in einer Membran orientiert eingeschlossen und auf einem Substrat aufgebracht ist. Zur Stabilisierung der orientiert angeordneten Moleküle ist diese mit einem Polymer-Film bedeckt (Japanische Patentanmeldung JP-A-63241432.

Weiterhin ist eine Anordnung bekannt, bei der die orientierten Membranmoleküle durch Zusatz bestimmter Substanzen und mit Hilfe bestimmter Verfahren, wie z. B. der Elektrophorese, miteinander vernetzt werden (EP-A-0 417 541). In diesem Fall sind die Membranen, die aus einer oder mehreren Proteinschichten bestehen können, ohne eine besondere Abdekkung stabil.

Der Nachteil dieser Anordnung besteht darin, daß die Orientierung der Protein-Moleküle nur mit Hilfe besonderer Vorrichtungen und Verfahren möglich ist. Nachteilig wirkt sich auch aus, daß bei den Anordnungen das verwendete Protein in hochgereinigter Form vorliegen muß.

Der Erfindung liegt die Aufgabe zugrunde, den Prozeß der Orientierung und Stabilisierung von strahlungsempfindlichen biologischen Substanzen in bioelektrischen Anordnungen zu vereinfachen, die Verwendung extrem hochgereinigten biologischen Materials zu vermeiden und Strahlungsenergie durch biologisches Material in einfachster Form in elektrische Energie umzusetzen.

Erfindungsgemäß wird diese Aufgabe durch die techniche Lehre des Anspruches 1 gelöst.

Danach werden als strahlungsempfindliches biologisches Material rhodopsinhaltige Mikroorganismen oder Zellen verwendet. Die rhodopsinhaltigen Mikroorganismen oder Zellen sind zweckmäßig in mindestens einer Membran eingebettet. Die Membran kann z. B. aus Agar-Agar, Calcium-Alginat oder aus organischen Polymeren bestehen. Die Mikroorganismen oder Zellen können statt in einer Membran auch in einem lyotropen Flüssigkristall eingebettet sein.

Es wurde überraschend gefunden, daß durch die Verwendung von rhodopsinhaltigen Mikroorganismen, wie Bakterien, oder rhodopsinhaltigen Zellen der gleiche strahlungselektrische Effekt erreicht wird, wie bei Verwendung von hochgereinigtem Rhodopsin, wie es heute üblich ist.

Die Erfindung soll in Ausführungsbeispielen erläutert werden. Es zeigen:
- Fig. 1 und 2: den Aufbau einer Meßanordnung
- Fig. 3 und 4: Meßergebnisse mit Halobakterien im lyotropen Flüssigkristall
- Fig. 5: Meßergebnisse mit Halobakterien in Agar-Agar

Es ist zunächst der Agar hergestellt worden (Medium 97 DSM-Katalog). Der Agar wird bei ca. 50° C geschmolzen. In zwei Milliliter des flüssigen Agars werden die Halobakterien (Halobacterium halobium, DSM-Nr. 670) suspendiert (230 mg Trockenmasse).

Der noch flüssige Agar mit den Bakterien wird in ein Plastikröhrchen 1 gegossen. Dieses wird anschließend fest verschlossen. Durch die Verschlüsse sind Platinelektroden 2 geführt, die an ein Kabel 7 angeschlossen sind. Nach Abkühlen auf ca. 20° C ist der Agar erstarrt. Dann erfolgen die Messungen mit dieser Probenanordnung 5.

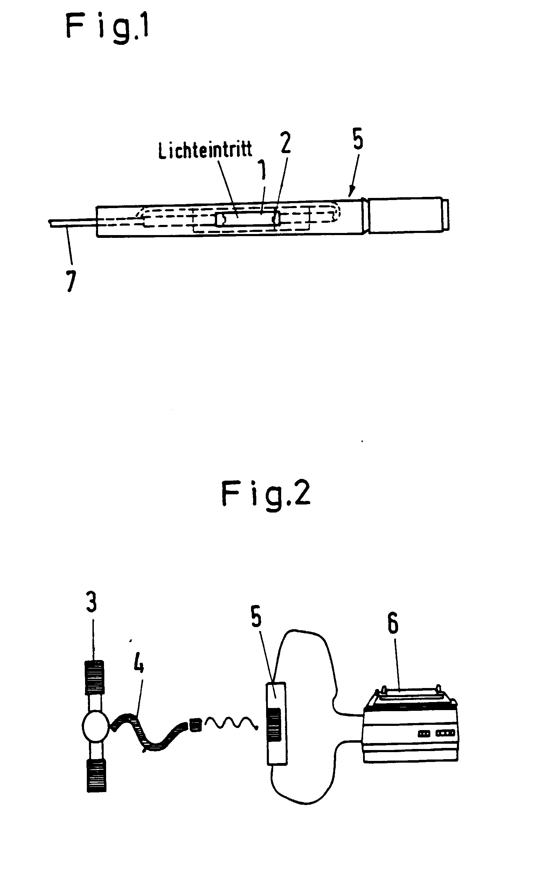

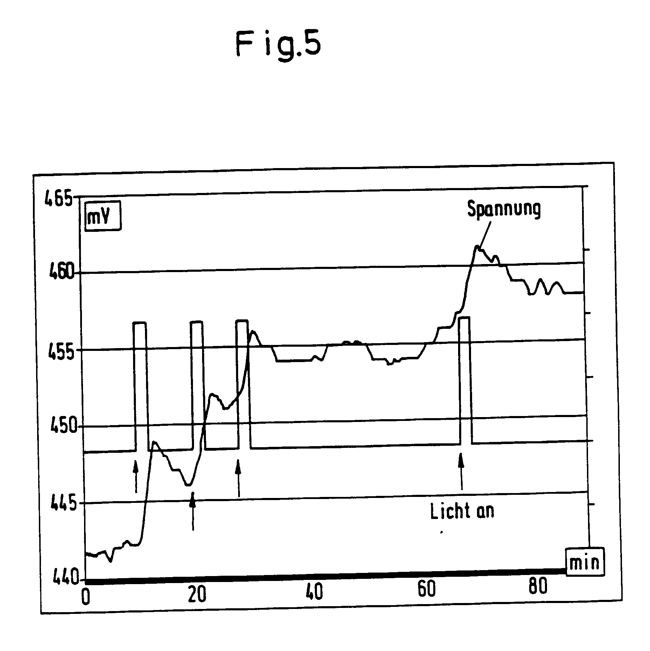

Der in der Fig. 2 schematisch dargestellte Meßaufbau hat eine Xenon-Kurzbogenlampe 3 und ein Glasfaser-Lichtleitkabel 4, mit dessen Hilfe die Probenanordnung 5 bestrahlt wird. Nach einer Belichtungszeit von ca. einer Minute wird eine Spannungsänderung hervorgerufen, die auf einem Schreiber 6 aufgezeichnet und in Fig. 5 dargestellt worden ist.

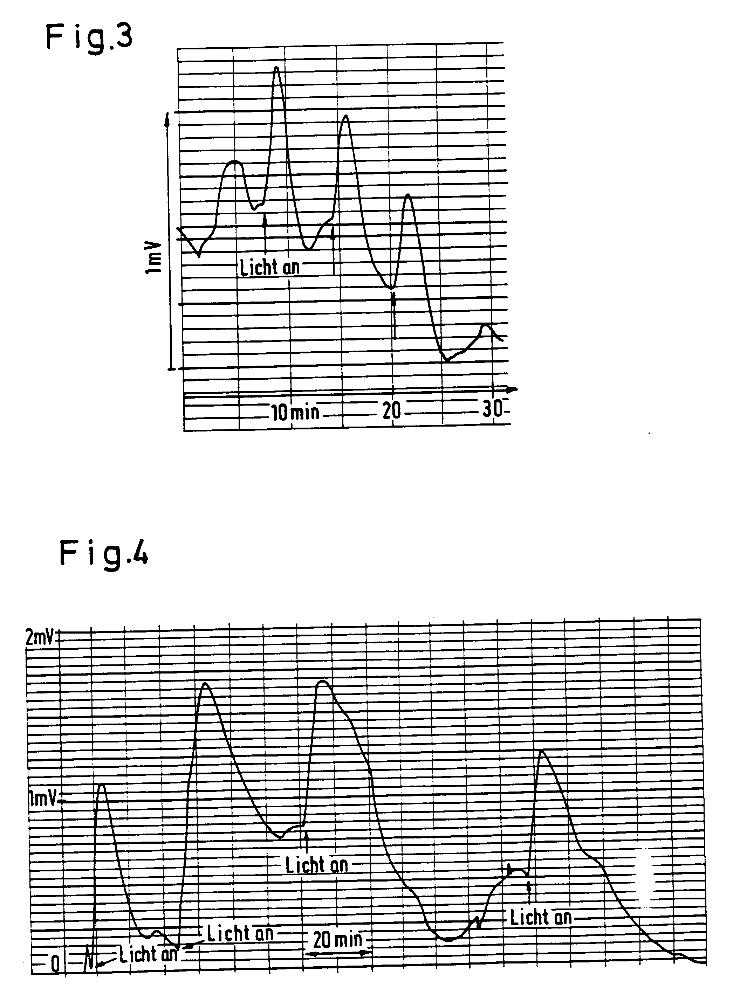

In einem weiteren Ausführungsbeispiel wird ein Gramm Brij 35 (Polyoxyethylenglykolmonolaurylether) bei ca. 40° C geschmolzen und mit einem Milliliter einer Suspension von Halobakterium halobium (130 mg Trockenmasse) verrührt. Beim Erstarren bildet sich der lyotrope Flüssigkristall aus. Dieser ist wie im ersten Beispiel vermessen worden (Belichtungszeit je eine Minute). Die Meßergebnisse sind in den Figuren 3 und 4 dargestellt.

## Patentansprüche

1. Strahlungsempfindliche bioelektrische Anordnung die ein strahlungsempfindliches biologisches Material verwendet,
**dadurch gekennzeichnet,**
daß das strahlungsempfindliche biologische Material aus rhodopsinhaltigen Mikroorganismen oder Zellen besteht , die in mindestens einer Membran eingebettet sind.

2. Anordnung nach Anspruch 1, **dadurch gekennzeichnet,** daß die Membran aus Agar-Agar besteht.

3. Anordnung nach Anspruch 1, **dadurch gekennzeichnet,** daß die Membran aus Calcium-Alginat besteht.

4. Anordnung nach Anspruch 1, **dadurch gekennzeichnet,** daß die Membran aus organischen Polymeren besteht.

## Claims

1. Radiation sensitive bioelectrical arrangement which uses a radiation-sensitive biological material, characterised in that the radiation-sensitive biological material consists of rhodopsin-containing micro-organisms or cells which are embedded in at least one membrane.

2. Arrangement according to claim 1 characterised in that the membrane consists of agar-agar.

3. Arrangement according to claim 1 characterised in that the membrane consists of calcium alginate.

4. Arrangement according to claim 1 characterised in that the membrane consists of organic polymers.

## Revendications

1. Dispositif bioélectrique sensible aux radiations qui utilise un matériau biologique sensible aux radiations, **caractérisé en ce que**
le matériau biologique sensible aux radiations consiste en cellules ou microorganismes contenant de la rhodopsine, qui est incorporée dans au moins une membrane.

2. Dispositif selon la revendication 1, **caractérisé en ce que** la membrane consiste en agar-agar.

3. Dispositif selon la revendication 1, **caractérisé en ce que** la membrane consiste en alginate-calcium.

4. Dispositif selon la revendication 1, **caractérisé en ce que** la membrane consiste en polymères organiques.
